# EUROPEAN PATENT APPLICATION

(11) **EP 1 609 404 A1**
(43) Date of publication of application: **28.12.2005**
(21) Application number: 04723351.5
(22) Date of filing: 25.03.2004
(51) Int. Cl.: A61B 1/00

(54) **CAPSULE TYPE ENDOSCOPE**

(30) Priority: 28.03.2003 JP 2003091882
(71) Applicant: Olympus Corporation, Tokyo 151-0072 (JP)
(72) Inventor: FUJIMORI, Noriyuki, Shibuya-ku, Tokyo 151-0072 (JP); YOSHIZAWA, Fukashi, Shibuya-ku, Tokyo 151-0072 (JP); YOSHIDA, Taishin, Shibuya-ku, Tokyo 151-0072 (JP)
(74) Representative: Winter, Brandl, Fürniss, Hübner Röss, Kaiser, Polte Partnerschaft Patent- und Rechtsanwaltskanzlei
(86) International application number: PCT/JP2004/004157
(87) International publication number: WO 2004/086956

(57) **Abstract**

A capsule endoscope which can make reliable inspection or diagnosis or the like at any time while securing a higher safety by controlling such as to interrupt the supply of power from a power supply when any inconvenience or abnormality or the like has occurred in the internal electric circuit system of the capsule endoscope in the state that the capsule endoscope has been introduced into the body cavity. In at least one of internal electric circuits which form an image pickup unit 11, a signal processing unit 12, a communication unit 13 and an illumination unit 15 arranged in the capsule endoscope are provided a temperature detection circuit 11a which detects an internal temperature, converts information indicating the detected temperature into an electric signal in a predetermined form and then outputs the signal, a comparator 11b which receives the signal from the temperature detection circuit 11a and make predetermined determination based on the received signal, and an internal-circuit current controller 11c which controls the supply of power to the internal electric circuit based on the result of determination by the comparator 11b.

## Description

### Technical Field

The present invention relates to a capsule endoscope shaped like a tablet, the capsule endoscope including a photographing optical system and an image pickup element.

### Background Art

In examinations of, for example, a body cavity, endoscope systems have been generally in practical use and have been widespread. Various devices such as an endoscope, a light source, an image processor, and a display device are assembled into the endoscope system.

The endoscope includes, for example, a tubular insertion portion having an image pickup element at the distal end thereof and an operation portion connected to the insertion portion. A universal cord extending from the insertion portion is connected to the light source and the image processor. The insertion portion is inserted into, for example, the mouth of a subject and is advanced through the body cavity thereof to observe a desired region.

The light source has a lamp. The illumination light of the lamp is supplied to the endoscope. The image processor has a drive circuit for driving the image pickup element and an image processing circuit for producing video signals from electric signals photoelectrically converted through the image pickup element. The display receives the video signals produced by the image processing circuit in the image processor and displays an endoscopic image on a screen.

In the conventional endoscope system, the length of the insertion portion to be inserted into the body cavity is limited. Thus, the range of observation and examination is also limited.

For example, Japanese Unexamined Patent Application Publication No. 2001-95755 discloses a capsule endoscope system and includes various proposals. According to Japanese Unexamined Patent Application Publication No. 2001-95755, the capsule endoscope system primarily includes a small endoscope, namely, a capsule endoscope and a receiving and recording device. The capsule endoscope has image pickup means including a photographing optical system, illumination means, communication means, and power supply means and the like. The respective components are contained in a capsule-shaped casing. The receiving and recording device has communication means for communicating with the capsule endoscope by radio, and recording means for recording a received signal.

According to the above-mentioned capsule endoscope system, for example, a subject swallows the capsule endoscope to introduce the capsule endoscope into their body cavity, so that the body cavity can be observed and examined. Advantageously, the capsule endoscope relatively easily achieves the observation and examination of a deep organ such as the small intestine. Generally, it is hard to observe and examine the small intestine using a conventional endoscope having the insertion portion.

In use of the capsule endoscope, a power switch arranged in a predetermined position in the capsule endoscope is turned on and is then swallowed by a subject, thus starting endoscopy. The swallowed capsule endoscope advances through the body cavity of the subject by peristaltic motion of the gut and reaches a predetermined region to be observed and examined. Finally, the capsule endoscope is evacuated naturally.

In a structure of the capsule endoscope disclosed by the Japanese Unexamined Patent Application Publication No. 2001-95755, however, its power switch cannot be returned to OFF state if once turned on. It also fails to disclose means to control the state of the power supply.

In other words, with the capsule endoscope disclosed by the above Patent Application Publication, if a subject has swallowed the capsule endoscope having the power switch turned on, it is by no means possible to thereafter turn off the power supply.

Hence, it might be possible that the power of the power supply is excessively supplied to an internal electric circuit, for example, if liquid and the like enter in a casing of the capsule endoscope or any trouble occurs in the internal electric circuit after the power switch of the capsule endoscope has been turned on, and the capsule endoscope has been introduced into the body cavity. On such occasion, it comes about that the power of the power supply is consumed before the capsule endoscope reaches the desired position for observation, for example, as the result of a heavily increased consumption of the power. As a consequence, such problem occurs that observation or inspection cannot be made as desired.

Meanwhile, it is well known that there is a tendency with the image pickup means such as usual image pickup element that the environment for use thereof is the one under higher temperature, more noise components and the like are caused in the image signal obtained. Therefore, in case any video signal produced based on an image signal obtained by an image pickup element under high temperature environment has been displayed on a display device, there is such problem that deterioration of the image quality can not be avoided.

Incidentally, in case the power is excessively supplied to the internal electric circuit of the capsule endoscope, it comes about that internal temperature of the capsule endoscope rises due to the cause as mentioned above, thereby to deteriorate the environment for use of the image pickup element. Then, further problem occurs that any endoscope image excellent in image quality can not be obtained from the image signal obtained at this time by the reasons as mentioned above.

Therefore, the present invention is to provide a capsule endoscope securing a higher safety by controlling for automatically interrupting the supply of power from the power supply in case any inconvenience or abnormality or the like has occurred in the internal electric circuit of the capsule endoscope introduced into the body cavity.

Also, the present invention is to provide a capsule endoscope which can avoid from obtaining in vain any image deteriorated in quality due to any inconvenience or the like occurred in the internal electric circuit system of the capsule endoscope inserted in the body cavity, thereby to make inspection or diagnosis or the like reliably at any time.

### Disclosure of Invention

The present invention provides a capsule endoscope including: temperature detecting means which is arranged in at least one of internal electric circuits and which detects an internal temperature of the corresponding internal electric circuit, converts information indicating the detected temperature into an electric signal in a predetermined form, and outputs the electric signal, the internal electric circuits constituting an image pickup unit, a signal processing unit, a communication unit, and an illumination unit, respectively; temperature determining means for performing a predetermined determination on the basis of the electric signal outputted from the temperature detecting means; and power control means for controlling power supply to the corresponding internal electric circuit on the basis of the determination result obtained by the temperature determining means. When the temperature detecting means detects an increase in temperature, the temperature determining means determines that a trouble or an abnormal condition occurs in the corresponding internal electric circuit, so that the power control means performs a power-supply interruption control for interrupting the operation of the corresponding internal electric circuit. Thus, the capsule endoscope in the ON state, which is put in a body cavity, can be changed to unavailable mode.

### Brief Description of the Drawings

Fig. 1 is a block diagram showing schematically the structure of a capsule endoscope according to a first embodiment of the present invention;
Fig. 2 is a block diagram showing schematically the structure of means for controlling a circuit power supply portion serving as a part of an internal structure constituting an image pickup unit in the capsule endoscope in Fig. 1; and
Fig. 3 is a block diagram showing schematically the structure of a capsule endoscope according to a second embodiment of the present invention.

### Best Mode for Carrying Out the Invention

Hereinbelow, a description is given with reference to the drawings.

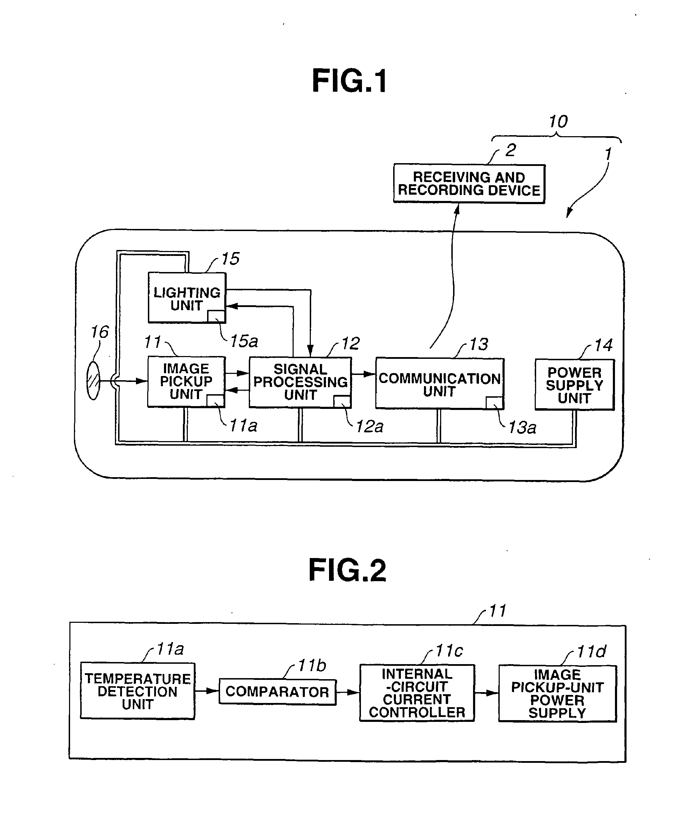

Referring to Figs. 1 and 2, a first embodiment of the present invention is described.

Referring to Fig. 1" a capsule endoscope system 10 has a capsule endoscope 1 and a receiving and recording device 2. The receiving and recording device 2 has a recording unit (not shown) for receiving image signals transmitted from the capsule endoscope 1 and recording the signals.

The capsule endoscope 1 includes an image pickup unit 11, a signal processing unit 12, a communication unit 13, a power supply unit 14, an illumination unit 15, and a photographing optical system 16.

The image pickup unit 11 functions as image pickup means and includes an image pickup element for receiving an image of an object, the image being formed by the photographing optical system 16, and performing predetermined photoelectric conversion to the image to generate predetermined image signals. The signal processing unit 12 serves as signal processing means for receiving the image signals outputted from the image pickup unit 11, performing various signal processing steps including a predetermined signal processing step to the received signals, and outputting the resultant image signals to the communication unit 13. The signal processing unit 12 also controls the illumination unit 15 and the image pickup unit 11. In other words, the signal processing unit 12 functions as a control unit for systematically controlling electric circuits included in the respective units of the capsule endoscope 1. The communication unit 13 functions as communication means for receiving the image signals generated from the signal processing unit 12 and for transmitting the signals to the receiving and recording device 2, arranged on the outside of the body of the subject, using predetermined radio communication means. The power supply unit 14 serves as power supply means for supplying power to the internal electric circuits, which are included in the image pickup unit 11, the signal processing unit 12, the communication unit 13, and the illumination unit 15, respectively. The power supply unit 14 supplies power to each unit by a predetermined amount required for the internal electric circuit included in the unit. The illumination unit 15 serves as illumination means for illuminating an object such as a digestive organ. The photographing optical system 16 forms the image of the object illuminated by the illumination unit 15.

The image pickup unit 11 has the image pickup element for performing photoelectric conversion and the electric circuit including a plurality of electric components. The electric circuit performs the predetermined signal processing step to image signals serving as electric signals indicating the image. The electric signals are obtained by the photoelectric conversion through the image pickup element.

The signal processing unit 12 has the electric circuit comprising a semiconductor circuit with a one-chip design. As mentioned above, the signal processing unit 12 performs various image signal processing steps and drive controls.

The communication unit 13 includes the electric circuit for receiving signals generated from the signal processing unit 12 and for transmitting the signals to the receiving and recording device 2.

The power supply unit 14 has the electric circuit comprising a plurality of electric components including a battery to supply a predetermined power to the respective electric circuits.

The illumination unit 15 includes the electric circuit comprising a plurality of electric components such as light emitting diodes.

The photographing optical system 16 includes, for example, a plurality of optical lenses and predetermined drive units for driving the optical lenses. The photographing optical system 16 converges a light beam, reflected from the object illuminated by the illumination unit 15, and forms the image of the object in a predetermined position, for example, on the image formation surface of the image pickup element in the image pickup unit 11.

In the capsule endoscope 1, each internal electric circuit constituting each unit has a temperature detection circuit and predetermined means for controlling a circuit power supply included in the internal electric circuit. In other words, referring to Figs. 1 and 2, the internal electric circuit constituting the image pickup unit 11 has a temperature detection circuit 11a and an image pickup-unit power supply 11d, which will be described later. The internal electric circuit of the signal processing unit 12 has a temperature detection circuit 12a and predetermined means for controlling a power supply for the signal processing unit 12. The internal electric circuit of the communication unit 13 has a temperature detection circuit 13a and predetermined means for controlling a power supply for the communication unit 13. The internal electric circuit of the illumination unit 15 has a temperature detection circuit 15a and predetermined means for controlling a power supply for the illumination unit 15.

Each predetermined means, controlling the corresponding circuit power supply in the corresponding internal electric circuit, performs a predetermined determination on the basis of temperature information serving as a signal indicative of a temperature detected by the corresponding temperature detection circuit, 11a, 12a, 13a, 15a, and then performs a control action based on the determination result.

The schematic structure of the predetermined means for controlling the corresponding circuit power supply in the corresponding internal electric circuit will now be described with reference to Fig. 2. Fig. 2 shows the image pickup unit 11 as an example.

As mentioned above, the image pickup unit 11 has the image pickup element and the electric circuit. The image pickup unit 11 further includes the image pickup-unit power supply 11d and an internal-circuit current controller 11c for controlling current flowing through the internal electric circuit constituting the image pickup unit 11.

The image pickup-unit power supply 11d receives power from the power supply unit 14 in Fig. 1, and properly supplies the power to the electric circuit constituting the image pickup unit 11 by the required amount. The internal-circuit current controller 11c controls the image pickup-unit power supply 11d.

Means for controlling the image pickup-unit power supply 11d includes the temperature detection circuit 11a, a comparator 11b serving as temperature determination means, and the internal-circuit current controller 11c serving as power control means.

The temperature detection circuit 11a detects an internal or ambient temperature of the image pickup unit 11, converts information indicating the detected temperature into an electric signal in a predetermined form, and then generates the signal. The comparator 11b receives the signal from the temperature detection circuit 11a, compares the signal with a predetermined value as a reference temperature that is preset, and determines whether the detected temperature is higher than the predetermined value. The comparator 11b then outputs a predetermined signal indicating the determination result to the internal-circuit current controller 11c. On the basis of the signal generated from the comparator 11b and related to the determination result, the internal-circuit current controller 11c controls the operation of the image pickup-unit power supply 11d when the signal is equal to the predetermined value. For example, the internal-circuit current controller 11c allows the image pickup-unit power supply 11d to interrupt power supply.

The temperature detection circuit 11a includes a resistor or a semiconductor circuit, which includes a diode, a bipolar transistor, or a metal-oxide-semiconductor transistor (MOS FET). The resistance of the resistor varies depending on, for example, temperature. Alternatively, the characteristics of the transistor vary depending on temperature. An output value of the temperature detection circuit 11a is generated as a temperature detection signal in a predetermined form.

The temperature detection circuits 11a, 12a, 13a, and 15a each include a temperature detection device (not shown). The temperature detection devices are arranged in the internal electric circuits constituting the image pickup unit 11, the signal processing unit 12, the communication unit 13, and the illumination unit 15 in the capsule endoscope 1, respectively. Alternatively, the temperature detection device is included in at least one of the internal electric circuits.

The temperature detection device includes a thermistor. The thermistor is integrated with each of the image pickup element (not shown) included in the image pickup unit 11, a processing circuit element (not shown) included in the signal processing unit 12, a communication element (not shown) included in the communication unit 13, and an illumination element (not shown) included in the illumination unit 15.

As mentioned above, though schematic representations of the detailed structures of the respective internal electric circuits included in the signal processing unit 12, the communication unit 13, and the illumination unit 15 are not shown, the structure of each internal electric circuit is substantially the same as that of the above-mentioned image pickup unit 11. In other words, the internal electric circuit includes a circuit power supply for the corresponding unit, the temperature detection circuits 12a, 13a, and 15a, a comparator 11b, and an internal-circuit current controller 11c.

The capsule endoscope 1 with the above configuration is used in combination with the receiving and recording device 2 having the recording unit for receiving image signals, captured and transmitted through the capsule endoscope 1, and for recording the signals. Thus, the image signals for observation of a desired region in the body cavity can be obtained and be recorded.

The image signals obtained in this manner are output to, for example, a predetermined display to display an image corresponding to the image signals. The image is observed, so that examination and diagnosis can be made on the basis of the observation.

The operation of the capsule endoscope 1 will now be described hereinbelow.

In examination using the capsule endoscope 1, the subject first swallows the capsule endoscope 1. Before the subject swallows the capsule endoscope 1, a predetermined power switch is turned on, thus activating the capsule endoscope 1. The swallowed capsule endoscope 1 advances through the body cavity of the subject by peristaltic motion and reaches an object region to be observed and examined.

The body cavity is illuminated by the illumination unit 15 in the capsule endoscope 1 under operating conditions. Consequently, the image of the illuminated object is formed on the image formation surface of the image pickup unit 11 through the photographing optical system 16. The image pickup unit 11 performs predetermined photoelectric conversion to the optical image formed on the image formation surface. Thus, image signals corresponding to the image of the object are output to the signal processing unit 12.

The signal processing unit 12 performs various signal processing steps to the supplied image signals. The resultant image signals are output to the communication unit 13. The communication unit 13 transmits the image signals to the receiving and recording device 2 provided in combination with the capsule endoscope 1. When receiving the image signals, the receiving and recording device 2 converts the signals into signals in the optimum form for recording and then records the converted signals into a predetermined recording medium (not shown) included therein.

On the other hand, when the capsule endoscope 1 is turned on, the temperature detection circuits 11a, 12a, 13a, and 15a each start the execution of the temperature detecting operation. The circuits 11a, 12a, 13a, and 15a are integrated with the image pickup unit 11, the signal processing unit 12, the communication unit 13, and the illumination unit 15, respectively.

The temperature detection circuits 11a, 12a, 13a, and 15a each detect a temperature to produce a detection signal, namely, a signal indicating temperature information of the corresponding unit. The detection signal is output from each temperature detection circuit to the corresponding comparator. For example, in the image pickup unit 11, the detection signal is generated from the temperature detection circuit 11a to the comparator 11b. Each comparator compares the signal, supplied from the corresponding temperature detection circuit, with the predetermined value. For example, the comparator 11b compares the signal supplied from the temperature detection circuit 11a with the predetermined value. Each comparator generates the comparison result to the corresponding internal-circuit current controller. For instance, in the image pickup unit 11, the comparator 11b outputs the comparison result to the internal-circuit current controller 11c.

Each internal-circuit current controller 11c, and the like conducts a predetermined determination process on the basis of the comparison result supplied from the corresponding comparator 11b, and the like.

When receiving the comparison result indicating that the output value of the corresponding temperature detection circuit 11a, 12a, 13a and 15a is higher than each reference temperature, each internal-circuit current controller performs a predetermined control process to stop the operation of the corresponding internal electric circuit. In other words, the internal-circuit current controller controls the corresponding power supply to interrupt power supply to the corresponding internal electric circuit among the image pickup unit 11, the signal processing unit 12, the communication unit 13 and the illumination unit 15. The control is called a power-supply interruption control (also referred to as an OFF control). For example, in the image pickup unit 11, when the output value of the temperature detection circuit 11a is higher than the reference value, the internal-circuit current controller 11c controls the image pickup-unit power supply 11d to interrupt power supply to the corresponding internal electric circuit.

In this case, the internal-circuit current controller 11c, and the like determines that the corresponding internal electric circuit has a trouble or an abnormal condition, and then performs the control process to interrupt the operation of the corresponding internal electric circuit. Thus, the capsule endoscope 1 is unavailable.

During the execution of the above-mentioned series of operations, the capsule endoscope 1 advances through the body cavity by peristaltic motion of the gut as mentioned above. Finally, the capsule endoscope 1 is naturally evacuated from the body cavity with safety and reliability.

As mentioned above, in the capsule endoscope 1 according to the present embodiment, a temperature of each internal electric circuit is detected. When the detected temperature is higher than the reference value, it is determined that the internal electric circuit has a trouble or an abnormal condition. Then, the power-supply interruption control for interrupting power supply to the internal electric circuit is automatically executed, so that the operation of the capsule endoscope 1 can be stopped.

In this case, the capsule endoscope 1 in the OFF state is naturally evacuated from the body cavity.

Therefore, if it is determined that any of the internal electric circuits included in the capsule endoscope 1 has a trouble or abnormal condition after putting the capsule endoscope 1 in the ON state into the body cavity of the subject, an oversupply of power to each internal electric circuit is interrupted to stop the operation of the capsule endoscope 1, thus ensuring higher safety.

In the present capsule endoscope 1, each internal electric circuit has the temperature detection circuit. Thus, temperatures of the respective internal electric circuits can be detected with higher accuracy. Moreover, a predetermined reference temperature can be specified in each internal electric circuit. Consequently, control with more detailed settings can be easily performed.

For example, if a temperature in the image pickup unit 11 increases, captured image signals may include a large amount of noise components. In this case, the temperature detection circuit 11a detects the increase in temperature of the image pickup unit 11, determines that the electric circuit included in the image pickup unit 11 has a trouble or an abnormal condition, and performs the power-supply interruption control to stop the operation of the image pickup unit 11.

Consequently, image signals having a deteriorated image quality caused by the trouble or abnormal condition in the image pickup unit 11, for example, image signals including a large amount of noise components inappropriate for examination are prevented from being captured uselessly. Thus, observed images can always be obtained favorably.

According to the first embodiment, the temperature detection circuits 11a, 12a, 13a, and 15a are arranged as temperature detection means for detecting temperatures in the respective internal electric circuits. Instead of the temperature detection circuits 11a, 12a, 13a, and 15a, for example, current detection circuits for detecting a current supplied to the corresponding internal electric circuit may be used. When each internal electric circuit includes the current detection circuit, components of the internal electric circuit and the corresponding control process are modified in accordance with the modified configuration of the circuit.

In other words, each comparator, arranged subsequent to the current detection circuit, receives an output of the current detection circuit and compares a current signal with a reference current as a predetermined value in advance. The comparison result is supplied to the corresponding internal-circuit current controller. The internal-circuit current controller performs a determination process based on the comparison result supplied from the comparator.

The determination process will now be described.

When receiving the comparison result indicating that an output of the current detection circuit is abnormal in comparison with the predetermined reference current, the internal-circuit current controller performs a predetermined control process to stop the operation of the corresponding internal electric circuit included with the illumination unit 15, the image pickup unit 11, the signal processing unit 12 or the communication unit 13. The control process is the same as that of the above-mentioned first embodiment, namely, the power-supply interruption control for controlling the circuit power supply.

As mentioned above, in the use of the current detection circuits for directly detecting a current flowing through the corresponding internal electric circuit instead of the temperature detection circuits according to the first embodiment, when the detection result of the current flowing through the internal electric circuit constituting, for example, the illumination unit indicates zero, it can be determined that the illumination operation is not performed. Because the illumination unit has a trouble, for example, an illumination light source member is unavailable in spite of the fact that power is supplied through the corresponding circuit power supply.

In other words, even when the photographing operation is normally performed by the image pickup unit, so long as the illumination operation through the illumination unit is not performed in the body cavity, it is hard to capture image signals used to obtain the optimum image for examination and diagnosis.

In this case, therefore, power supply to at least the illumination unit is immediately shut off to stop the operation of the capsule endoscope 1. The capsule endoscope 1 in the OFF state is naturally evacuated. Consequently, the useless operation for capturing image signals inappropriate for examination using the capsule endoscope 1 with a trouble can be interrupted. The safety against a trouble or an abnormal condition of each internal electric circuit can be increased.

When the increase in current of power supplied to any internal electric circuit is detected, it is determined that the internal electric circuit has a trouble or an abnormal condition, in the same way as the detection of the increase in temperature in the foregoing first embodiment. As mentioned above, when the current detection circuits are used instead of the temperature detection circuits, the same advantages as those of the first embodiment can be obtained.

According to the foregoing embodiment, the detection signals of the temperature detection circuits 11a, 12a, 13a, and 15a in the respective internal electric circuits are supplied to the corresponding comparators, respectively. Each comparator compares the received signal with the predetermined reference temperature. The comparison result is supplied to the corresponding internal-circuit current controller. The internal-circuit current controller performs the predetermined control process.

Different from the above embodiment, the respective detection signals of the temperature detection circuits 11a, 12a, 13a, and 15a can be transmitted to the receiving and recording device 2 through the communication unit 13.

In this structure, the receiving and recording device 2 includes means corresponding to the comparator 11b and means corresponding to the internal-circuit current controller 11c. Accordingly, in the receiving and recording device 2, whether each internal electric circuit in the capsule endoscope 1 has a trouble or an abnormal condition is determined on the basis of the corresponding detection signal transmitted from the communication unit 13. A predetermined control signal based on the determination result may be transmitted to the corresponding internal electric circuit through the communication unit 13 in the capsule endoscope 1.

Using the structure, the respective internal electric circuits in the capsule endoscope 1 can be easily simplified. Thus, the capsule endoscope 1 can be further miniaturized, resulting in the reduction in manufacturing cost.

According to the first embodiment, the power supply unit 14 is arranged in the capsule endoscope 1. The placement of the power supply unit 14 is not limited to the interior of the capsule endoscope 1. For example, the power supply unit 14 can be placed in the exterior of the capsule endoscope 1, namely, a device which is not inserted into the body cavity and is used in the outside of the body. That is, the power supply unit 14 may be provided for the receiving and recording device 2 or an external power supply device, which is arranged separately from the receiving and recording device 2 and includes a predetermined power supply circuit. Power is supplied from the above-mentioned device through predetermined radio communication means. In other words, a so-called wireless power supply method can be used.

In this case, the capsule endoscope 1 includes a receiving unit having means for receiving power supplied from the above-mentioned external power supply device in a wireless manner and for distributing the received power to the respective internal electric circuits in the capsule endoscope 1. Thus, power supplied from the external power supply device in a wireless manner can be distributed to the respective internal electric circuits in the capsule endoscope 1 through the receiving unit.

When the occurrence of a trouble or an abnormal condition in any internal electric circuit in the capsule endoscope 1 is detected by the corresponding detection means such as the temperature detection circuit or the current detection circuit, power supply to the internal electric circuit is shut off and the reception of power through the receiving unit is also interrupted by the same action as that of the foregoing embodiment. In this manner, the operation of the capsule endoscope 1 is interrupted, thus obtaining the same advantages as those of the above embodiment.

Further, when the occurrence of a trouble or an abnormal condition in any internal electric circuit is detected in the capsule endoscope 1 with the structure according to the wireless power supply method, the signal processing unit 12 can communicate with the external power supply device through the communication unit 13 or the receiving unit so that the predetermined power-supply interruption control is performed in order to shut off power supply.

As mentioned above, the capsule endoscope 1, which receives power from the external power supply device in a wireless manner to drive the respective internal electric circuits, is controlled so that continuously-supplied power is shut off in predetermined cases. Thus, the same advantages as those of the above embodiment can be obtained.

On the other hand, according to the above embodiment, if a trouble or an abnormal condition is detected in any internal electric circuit of the capsule endoscope 1, only the control for interrupting power supply is performed. In addition, simultaneously with the power-supply interruption control, an abnormal information signal as a predetermined signal indicating the occurrence of a trouble or an abnormal condition in the internal electric circuit can be formed by the signal processing unit 12. The signal is transmitted from the signal processing unit 12 to the receiving and recording device 2 through the communication unit 13.

In this case, as means for informing the operator or the subject of the occurrence of the trouble or abnormal condition in response to the reception of the abnormal information signal through the receiving and recording device 2, predetermined announcement means such as a buzzer or a lighting or flashing device using a light emitting diode can be provided for the receiving and recording device 2.

In this structure, the operator or the subject can immediately notice the occurrence of a trouble in the capsule endoscope 1 put in the body cavity. For example, just after the announcement of the trouble, the subject again swallows another capsule endoscope 1, so that the examination can be smoothly continued without being interrupted.

In this case, the capsule endoscope 1 which has already been put into the body cavity and may have a trouble or abnormal condition is subjected to the predetermined power-supply interruption control in the same way as the above embodiment. Therefore, the capsule endoscope 1 in the OFF state is naturally evacuated.

The arrangement of the above-mentioned announcement means may produce the following advantages.

When the increase in temperature of the image pickup unit 11 is detected by the temperature detection circuit 11a and the occurrence of a trouble or abnormal condition in the image pickup unit 11 is determined, the power-supply interruption control for interrupting the operation of the image pickup unit 11 is performed in the same way as the above embodiment. At that time, capturing image signals through the capsule endoscope 1 is suspended. In other words, if the image pickup unit 11 has any trouble or an abnormal condition, the use of the capsule endoscope 1 can be interrupted without uselessly capturing image signals with a large amount of noise components inappropriate for examination.

Simultaneously with the interruption, the above-mentioned announcement means can immediately inform the operator or the subject of the occurrence of the trouble or abnormal condition in any internal electric circuit. Accordingly, at this time, the subject again swallows another capsule endoscope 1, so that the same examination can be continued. Therefore, time required for the examination is not wasted, resulting in more efficient examination and diagnosis.

According to the above embodiment, in the capsule endoscope 1, the temperature detection circuits 15a, 11a, 12a, and 13a are integrated with the illumination unit 15, the image pickup unit 11, the signal processing unit 12, and the communication unit 13, respectively. The present invention is not limited to the above structure. The present invention can be applied to any structure so long as a temperature in the interior of the capsule endoscope 1 can be detected by at least one temperature detection circuit 12a.

Therefore; any one of the internal electric circuits integrally can include a temperature detection circuit. For example, only the signal processing unit 12 integrally may include the temperature detection circuit 12a. In the structure, the same advantages as those of the first embodiment can be obtained.

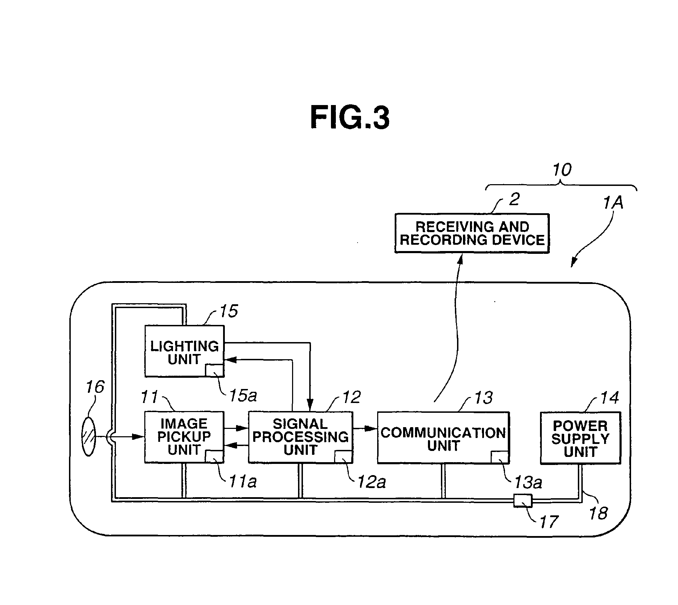

Fig. 3 shows a capsule endoscope 1A according to a second embodiment of the present invention.

Referring to Fig. 3, the capsule endoscope 1A has substantially the same structure as that of the capsule endoscope 1 according to the first embodiment. The capsule endoscope 1A differs from the capsule endoscope 1 in such a way that the capsule endoscope 1A includes a thermal fuse 17 in a power/signal line 18 serving as a part of internal electric circuits.

According to the second embodiment, the capsule endoscope 1A has the thermal fuse 17 on the power/signal line 18 near the power supply unit 14. The respective internal electric circuits of the capsule endoscope 1A are connected to the power supply unit 14 through the thermal fuse 17. The other arrangement and components are the same as those of the first embodiment. The components similar to those of the first embodiment are referenced using the same reference numerals to omit the description thereof.

According to the second embodiment, in the capsule endoscope 1A with the above-mentioned structure, if any trouble or an abnormal condition occurs in any of the internal electric circuits, the corresponding temperature detection circuit 11a, 12a, 13a, or 15a performs the power-supply interruption control. Moreover, for example, when the power supply unit 14 supplies an excess of power and current exceeds the rated value of the thermal fuse 17, the thermal fuse 17 is disconnected. Consequently, the power supply through the power supply unit 14 is completely shut off, resulting in the interruption of the operation of the capsule endoscope 1A.

According to the second embodiment, the same advantages as those of the first embodiment can be obtained. Further, the thermal fuse 17 is disconnected in the excess power supply mode of the power supply unit 14, so that the power supply from the power supply unit 14 to the internal electric circuits is interrupted. Thus, the power supply interruption control can be performed with more reliability.

According to the second embodiment, the thermal fuse 17 is arranged in the power/signal line 18. In place of the thermal fuse 17, for example, a thermistor serving as a resistor whose resistance varies depending on temperature can be arranged. In the structure using the thermistor, the same advantages as those of the second embodiment can be obtained.

The structure according to the second embodiment can be applied to the modifications of the first embodiment. In other words, the capsule endoscope 1A can be applied to the current detection circuits used instead of the temperature detection circuits, one temperature detection circuit provided for any one of the internal electric circuits, power supplied from the external power supply device in a wireless manner, and the other modifications. In the above applied modifications, the same advantages as those of the first embodiment can be obtained.

### Industrial Applicability

As mentioned, the capsule endoscope of the present invention controls to automatically interrupt the supply of power from the power supply when any inconvenience or abnormality or the like has occurred in the internal electric circuit system in the state that the capsule endoscope has been introduced into the body cavity. Therefore, the present invention is adapted to make reliable inspection or diagnosis or the like at any time while securing a higher safety by avoiding from obtaining in vain any image deteriorated in quality due to inconvenience or the like.

## Claims

1. A capsule endoscope **characterized by** comprising:
temperature detection means which is arranged in at least one of internal electric circuits and which detects an internal temperature of the corresponding internal electric circuit, converts information indicating the detected temperature into an electric signal in a predetermined form, and generates the electric signal, the internal electric circuits constituting an image pickup unit, a signal processing unit, a communication unit, and an illumination unit, respectively;
temperature determination means for performing a predetermined determination on the basis of the electric signal generated from the temperature detection means; and
power control means for controlling power supply to the corresponding internal electric circuit on the basis of the determination result obtained by the temperature determination means.

2. The capsule endoscope according to Claim 1, **characterized in that** when the temperature determination means determines that the internal temperature is higher than a predetermined value, the power control means controls so that the power supply to the corresponding internal electric circuit is interrupted.

3. The capsule endoscope according to Claim 1, **characterized in that**
each internal electric circuit comprises a semiconductor device, and
the temperature detection means is integrated with the semiconductor device.

4. The capsule endoscope according to Claim 1, **characterized in that** the temperature detection means comprises a member which is independent of the internal electric circuits and is arranged in a power supply line constituting a part of the internal electric circuits.

5. The capsule endoscope according to Claim 4, **characterized in that** the temperature detection means includes a thermal fuse.

6. The capsule endoscope according to Claim 4, **characterized in that** the temperature detection means includes a thermistor.
